# EUROPÄISCHE PATENTSCHRIFT

(11) **EP 3 221 031 B1**
(45) Veröffentlichungstag und Bekanntmachung des Hinweises auf die Patenterteilung: **19.09.2018**
(21) Anmeldenummer: 15794893.6
(22) Anmeldetag: 12.11.2015
(51) Int. Cl.: B01D 53/86, B01J 23/42, B01J 23/44, B01J 23/46, B01J 23/52, C07C 7/148

(54) **ENTFERNUNG VON SAUERSTOFF AUS KOHLENWASSERSTOFF-HALTIGEN GASGEMISCHEN**
REMOVAL OF OXYGEN FROM HYDROCARBON-CONTAINING GAS MIXTURES
ÉLIMINATION DE L'OXYGÈNE DE MÉLANGES GAZEUX CONTENANT DES HYDROCARBURES

(30) Priorität: 20.11.2014 DE 102014223759
(43) Veröffentlichungstag der Anmeldung: 27.09.2017
(73) Patentinhaber: Wacker Chemie AG, 81737 München (DE)
(72) Erfinder: SCHULTHEISS, Peter, 84489 Burghausen (DE); DAFINGER, Willibald, 94133 Röhrnbach (DE); ECKERT, Marc, Paducah, KY 42001 (US); FRANK, Friedrich, 84387 Julbach (DE)
(74) Vertreter: Ege, Markus
(86) Internationale Anmeldenummer: PCT/EP2015/076374
(87) Internationale Veröffentlichungsnummer: WO 2016/078993

(56) Entgegenhaltungen:
- WO-A1-2006/075025
- GB-A- 565 991
- US-A- 5 446 232
- US-A1- 2007 004 926
- US-A1- 2010 048 972

## Beschreibung

Die Erfindung betrifft Verfahren zur Entfernung von Sauerstoff aus Kohlenwasserstoff-haltigen Gasgemischen.

Kohlenwasserstoffe, wie Ethylen, Propylen oder sonstige Kohlenwasserstoffe mit 1 bis 7 Kohlenstoffatomen, sind Standardgrundstoffe der chemischen Industrie. Gase solcher Kohlenwasserstoffe enthalten oftmals Verunreinigungen, beispielsweise Sauerstoff, der bei der Verwertung der Kohlenwasserstoffgase hinderlich sein kann und in solchen Fällen zumindest teilweise aus den Kohlenwasserstoffgasen entfernt werden sollte. Ein gängiges Reinigungsverfahren ist die Kryodestillation. Für Sauerstoff enthaltende Kohlenwasserstoffgase ist dieses Verfahren aber problematisch, da sich solche Mischungen destillativ nicht hinreichend trennen lassen und es stattdessen beim Abdestillieren von Verunreinigungen zu einer Anreicherung von Sauerstoff kommen kann. Zudem kann es bei der Kryodestillation zu einer Cokondensation von Kohlenwasserstoffgasen und Sauerstoff kommen, was sicherheitstechnisch sehr problematisch ist. Eine Anreicherung von Sauerstoff in Kohlenwasserstoffgasen gilt es tunlichst zu vermeiden, da Sauerstoff für viele Verwertungen von Kohlenwasserstoffgasen hinderlich ist und, besonders problematisch, instabile oder gar explosible Gasmischungen gebildet werden können. Auch beim Membranverfahren erfolgt keine ausreichende Trennung, was zu einer Anreicherung von Sauerstoff im Retentat bzw. Permeat und somit wiederum zur Bildung von explosionsfähigen sauerstoffhaltigen Gasgemischen führen kann.

Bekannt ist auch die oxidative Reinigung von Gasgemischen, bei der Kohlenwasserstoffe und Sauerstoff des Gasgemisches miteinander zur Reaktion gebracht werden unter Bildung nicht hinderlicher oder leicht abtrennbarer Produkte, wie Kohlendioxid und Wasser. Diese Umsetzungen sind allerdings stark exotherm. Die oxidative Reinigung hat vielfache Anwendung gefunden für Gasgemische, die sehr geringe Mengen an Kohlenwasserstoffen und/oder sehr geringe Mengen an Sauerstoff enthalten, wie beispielsweise Industrie- oder Autoabgase. Im Falle von Gasgemischen, die größere Anteile sowohl an Kohlenwasserstoffen als auch an Sauerstoff enthalten, werden bei der oxidativen Reinigung so erhebliche Wärmemengen frei, dass die Bildung unerwünschter Nebenprodukte bzw. Zerfallsprodukte oder gar ein explosiver Verlauf der Oxidationsreaktion zu erwarten ist.

Um die oxidative Reinigung bei niedrigeren Reaktionstemperaturen durchführen zu können, haben vielfach Katalysatoren Einsatz gefunden, beispielsweise Ruthenium-Katalysatoren. Aber auch Katalysatoren erfordern zum Erreichen ihrer katalytischen Aktivität bestimmte Mindesttemperaturen, die sogenannte "Light-off"-Temperatur, die im Falle von Gasgemischen mit hohen Gehalten an Kohlenwasserstoffen und Sauerstoff weiterhin in einem sicherheitstechnisch problematischen Bereich liegen.

So beschreibt beispielsweise die US 4093703 die oxidative Entfernung von Ethylen aus Gasgemischen, die bis zu 1,8 Vol.-% Ethylen enthalten. Zur Reinigung von Ethylen aus Crackern lehrt die US 2010/0048972 den Einsatz von Ruthenium-Katalysatoren. Die darin beschriebenen zu reinigenden Gase enthalten Sauerstoff im ppm-Bereich. Die US 2010/0048972 weist vom Einsatz von Palladium-Katalysatoren zur Sauerstoffentfernung weg. Die EP 0499087 befasst sich mit der Entfernung von Stickoxid-, Kohlenmonoxid- und Kohlenwasserstoff-Spuren aus Sauerstoff-reichen Abgasen von Gasturbinen. Die Abgase bestehen im Wesentlichen aus Stickstoff. Die GB 883945 beschreibt Verfahren zur Reinigung von Abgasen aus der oxidativen Umsetzung von Ammoniak zu Salpetersäure, bei der Stickoxide und Sauerstoff durch Zusatz von ungesättigten Verbindungen und Sulfiden entfernt werden. Auch diese Abgase bestehen im Wesentlichen aus Stickstoff. Van de Beld beschreibt in Chemical Engineering and Processing 34, (1995), Seiten 469 bis 478 die Totaloxidation von Ethen und Propan an Pd-Katalysatoren, die auf Al₂O₃ geträgert sind. Auch Rusu diskutiert in Environmental Engineering and Management Journal, 2003, Vol. 2, No. 4, Seiten 273 bis 302 die Oxidation von Ethen. Die am häufigsten eingesetzten Katalysator-Systeme sind hierbei Pd oder Pt auf TiO₂, Al₂O₃ oder SiO₂. Hosseini empfiehlt in Catalysis Today, 122, (2007), Seiten 391 bis 396 für die Totaloxidation von Propen Pd und Au auf TiO₂ als Katalysator. Aus der EP 2656904 sind Katalysatoren zur Reinigung von Abgasen aus Dieselmotoren bekannt. Die Katalysatoren enthalten eine katalytische Beschichtung mit Pt, Pd und einer kohlenstoffspeichernden Verbindung, wie Zeolith, sowie eine weitere Beschichtung mit Pd und Au.

Die WO 2014/006017 A1 offenbart ein Verfahren zur Entfernung von Sauerstoff aus Kohlenwasserstoffströmen unter Verwendung von Platin und Platin/Zinn Katalysatoren.

Weil die Handhabung und Reinigung von Kohlenwasserstoffgasen, die mit erheblichen Mengen an Sauerstoff verunreinigt sind, weiterhin Probleme bereiten, werden solche Gasgemische vielfach verbrannt, was aus okonomischer Sicht fatal ist, da Kohlenwasserstoffe, wie Ethylen, wertvolle petrochemische Ausgangsstoffe sind.

Vor diesem Hintergrund bestand die Aufgabe, Verfahren zur oxidativen Entfernung von Sauerstoff aus Gasgemischen bereitzustellen, die zum Großteil aus Kohlenwasserstoffen bestehen und erhebliche Anteile an Sauerstoff enthalten. Dabei sollten die oben genannten Probleme gelöst werden konnen und insbesondere die Bildung von Nebenprodukten minimiert und Explosionen unterbunden werden können. Nach Moglichkeit sollten bei kontinuierlicher Reaktionsfuhrung auch Kohlenwasserstoffgase mit im Laufe der Zeit variabler Gasgemische, insbesondere variablen Anteilen an Sauerstoff und/oder Kohlenwasserstoffen, oxidativ aufgereinigt werden konnen.

Gegenstand der Erfindung sind Verfahren zur Entfernung von Sauerstoff aus Kohlenwasserstoff-haltigen Gasgemischen, dadurch gekennzeichnet, dass
ein Kohlenwasserstoff-haltiges Gasgemisch enthaltend ≥ 50 Vol.-% an einem oder mehreren Kohlenwasserstoffen, 2 bis 10 Vol.-% Sauerstoff und gegebenenfalls ein oder mehrere Gase aus der Gruppe umfassend Stickstoff, Edelgase, Kohlendioxid, Kohlenmonoxid und Wasser,
in einen isotherm betriebenen Reaktor eingeführt wird,
in dem der im Kohlenwasserstoff-haltigen Gasgemisch enthaltene Sauerstoff in Gegenwart eines oder mehrerer Katalysatoren zumindest teilweise zu Kohlendioxid und Wasser umgesetzt wird, wobei das Kohlenwasserstoff-haltige Gasgemisch keinen Wasserstoff enthält und dem Kohlenwasserstoff-haltigen Gasgemisch kein Wasserstoff zugesetzt wird, und
wobei ein oder mehrere Katalysatoren ausgewählt werden aus der Gruppe umfassend Palladium/Gold, Platin/Gold und deren Salze, wobei sich die Angaben in Vol.-% auf das Gesamtvolumen des dem Reaktor zugeführten Kohlenwasserstoff-haltigen Gasgemisches beziehen und sich insgesamt auf 100 Vol.-% aufaddieren.

Der kombinierte Einsatz von Katalysatoren und isotherm betriebenen Reaktoren erlaubt überraschenderweise die kontrollierte oxidative Reinigung von Sauerstoff- und Kohlenwasserstoffreichen Gasgemischen.

Die Kohlenwasserstoff-haltigen Gasgemische werden im Folgenden abgekürzt auch als Gasgemische bezeichnet.

Im erfindungsgemäßen Verfahren werden im Allgemeinen Kohlenwasserstoffe des zu reinigenden Gasgemisches durch den im Gasgemisch als Verunreinigung enthaltenen Sauerstoff oxidativ umgesetzt, so dass der Sauerstoffgehalt im Gasgemisch verringert wird. Im Reaktor findet also im Allgemeinen eine Umsetzung von Sauerstoff mit Kohlenwasserstoffen zu Kohlendioxid und Wasser statt. Das Verfahren kann also als oxidative Selbstreinigung bezeichnet werden.

Die Kohlenwasserstoffe haben vorzugsweise 1 bis 7 Kohlenstoffatome, besonders bevorzugt 1 bis 5 Kohlenstoffatome und am meisten bevorzugt 1 bis 3 Kohlenstoffatome. Die Kohlenwasserstoffe liegen bei einem Druck von 1 bar_{abs.} vorzugsweise in Form eines Gases vor. Die Kohlenwasserstoffe haben bei einem Druck von 1 bar_{abs.} Siedepunkte von vorzugsweise ≤ -20°C, besonders bevorzugt ≤ -40°C und am meisten bevorzugt ≤ -60°C. Die Kohlenwasserstoffe können gesättigt oder vorzugsweise ethylenisch ungesättigt sein. Beispiele für gesättigte Kohlenwasserstoffe sind Methan, Ethan, Propan, Butan oder Pentan. Beispiele für ethylenisch ungesättigte Kohlenwasserstoffe sind Propen, Butadien, Acetylen und insbesondere Ethylen.

Die Kohlenwasserstoffe tragen vorzugsweise keine funktionellen Gruppen, wie Alkohol-Gruppen oder Halogenide, insbesondere keine Sauerstoffatome, Schwefelatome, Halogenatome oder Stickstoffatome enthaltende funktionelle Gruppen. Die Kohlenwasserstoffe bestehen vorzugsweise ausschließlich aus Kohlenstoffatomen und Wasserstoffatomen oder deren Isotope. Besonders bevorzugte Kohlenwasserstoffe sind ethylenisch ungesättigte Kohlenwasserstoffe, insbesondere Ethylen.

Beispiele für Edelgase sind Helium, Neon, Krypton, Xenon, Radon und insbesondere Argon.

Das zu reinigende Kohlenwasserstoff-haltige Gasgemisch, das in den isothermen Reaktor eingeführt wird, enthält ≥ 50 Vol.-%, vorzugsweise 60 bis 93,5 Vol.-%, besonders bevorzugt 65 bis 90 Vol.-%, noch mehr bevorzugt 65 bis 85 Vol.-% und am meisten bevorzugt 70 bis 80 Vol.-% an einem oder mehreren Kohlenwasserstoffen. Gesättigte Kohlenwasserstoffe, insbesondere Methan oder Ethan, sind hierbei vorzugsweise zu 0,1 bis 10 Vol.-% und besonders bevorzugt 3 bis 6 Vol.-% enthalten. Das Gasgemisch enthält Sauerstoff zu Anteilen von ≤ 10 Vol.-%, vorzugsweise ≤ 6,5 Vol.-%, besonders bevorzugt ≤ 5 Vol.-% und am meisten bevorzugt ≤ 4 Vol.-%. Sauerstoff ist zu Anteilen von ≥ 2 Vol.-%, vorzugsweise ≥ 3 Vol.-% und besonders bevorzugt ≥ 4 Vol.-% im Gasgemisch enthalten. Bei den verbleibenden Gasanteilen handelt es sich um die weiteren Gase ausgewählt aus der Gruppe umfassend Stickstoff, Edelgase, Kohlendioxid, Kohlenmonoxid und Wasser. Die Angaben in Vol.-% beziehen sich auf das Gesamtvolumen des jeweiligen Gasgemisches und addieren sich insgesamt auf 100 Vol.-% auf.

Die Kohlenwasserstoff-haltigen Gasgemische enthalten keinen Wasserstoff.

Als Reaktor kommt im Allgemeinen ein Rohrenreaktor oder Rohrbündelreaktor zum Einsatz. Vorzugsweise wird das Gasgemisch in Röhren, die von einem Kühlmittel umflossen werden, umgesetzt. Als Kühlmittel kommen beispielsweise Öl- oder Salzlösungen oder Wasser in Betracht. Bevorzugt ist die Siedewasserkühlung. Dadurch kann die Temperatur im Reaktor geregelt werden. Solche Reaktoren und deren Auslegung sind dem Fachmann vertraut.

Der Reaktor wird isotherm bzw. vorzugsweise nicht adiabatisch betrieben. Dies schließt jedoch nicht aus, dass es innerhalb des Reaktors, insbesondere vereinzelt oder lokal bzw. gelegentlich, zu sogenannten hot spots kommen kann. Hot spots sind lokale Überhitzungen bzw. Temperatur-Maxima, die sich aber während des Betriebs des Reaktors nivellieren. Die Temperatur des aus dem Reaktor austretenden Gasgemisches unterscheidet sich von der Temperatur des in den Reaktor eintretenden Gasgemisches um vorzugsweise ≤ 50°C, mehr bevorzugt ≤ 30°C, besonders bevorzugt ≤ 25°C, noch mehr bevorzugt ≤ 20°C und am meisten bevorzugt ≤ 15°C.

Der Reaktor ist im Allgemeinen mit einem oder mehreren Katalysatoren beschickt, beispielsweise als Festbettkatalysator. Die Katalysatoren enthalten im Allgemeinen Edelmetalle Palladium/Gold oder Platin/Gold oder deren Salze.

Die Edelmetall (salze) können auf Träger aufgebracht sein (Trägerkatalysatoren). Beispiele für Träger sind Oxide von Metallen oder Halbmetallen, wie Zircon, Tita, Cer, Lanthan oder insbesondere Aluminium oder Silicium. Besonders bevorzugt sind Silica, Aluminiumoxide oder Bentonit.

Des Weiteren können die Katalysatoren ein oder mehrere Dotierstoffe enthalten, beispielsweise anorganische Salze, wie Halogenide, Oxide, Nitrate, Nitrite, Silikate, Carbonate, Borate, Aluminate, Molybdate, Wolframate, Vanadate, Niobate, Tantalate, Titanate oder Zirkonate.

Die Katalysatoren konnen nach an sich bekannten Methoden hergestellt werden, wie beispielsweise in der DE 102006058800 A1, US 4093559 oder EP 565952 beschrieben.

Der Reaktor wird mit dem Gasgemisch beschickt. Der Reaktor wird vorzugsweise in kontinuierlicher Fahrweise betrieben. Das Gasgemisch kann mehrfach durch den Reaktor geführt werden. Es ist aber im Allgemeinen hinreichend, das Gasgemisch einmal durch den Reaktor zu führen.

Der Reaktor kann so ausgerichtet sein, dass die Strömungsrichtung des Gasgemisches im Reaktor im Wesentlichen horizontal oder vorzugsweise im Wesentlichen vertikal ist. Bei vertikaler Strömungsrichtung kann das Gasgemisch den Reaktor von oben nach unten oder vorzugsweise von unten nach oben durchströmen. Insbesondere die bevorzugten Varianten sind zur Lösung der erfindungsgemäßen Aufgabe und zur effizienteren Abfuhr der Wärme aus dem Reaktor besonders dienlich.

Den zu reinigenden Gasgemischen wird vorzugsweise kein Mittel zugesetzt, das zur Reinigung dient oder dienen könnte, insbesondere kein zusätzlicher Sauerstoff und/oder kein Reduktionsmittel, wie Kohlenwasserstoffe. Den Gasgemischen wird also vorzugsweise nichts zugesetzt, was von den nach den erfindungsgemäßen Verfahren zu reinigenden Kohlenwasserstoff-haltigen Gasgemischen verschieden ist.

Vorzugsweise wird in den Reaktor kein weiteres Gas und/oder kein weiteres Mittel eingeführt, insbesondere kein Sauerstoff. In den Reaktor wird während der Durchführung des erfindungsgemäßen Verfahrens also vorzugsweise ausschließlich das zu reinigende Gasgemisch eingebracht.

Das Gasgemisch hat bei Eintritt bzw. unmittelbar vor Eintritt in den Reaktor eine Temperatur von vorzugsweise ≤ 300°C, mehr bevorzugt ≤ 250°C, besonders bevorzugt ≤ 230°C, noch besonders bevorzugt ≤ 210°C und am meisten bevorzugt ≤ 200°C. Die Temperatur ist hierbei vorzugsweise ≥ 100°C, besonders bevorzugt ≥ 120°C, noch mehr bevorzugt ≥ 150°C und am meisten bevorzugt ≥ 180°C.

Das Gasgemisch, das dem Reaktor zugeführt wird, kann mit einem oder mehreren Wärmetauschern auf die gewünschte Temperatur gebracht werden. Beispiele für Wärmetauscher sind Plattenwarmetauscher, Rohr(bündel)wärmeüberträger, U-Rohr-Warmeuberträger, Mantelrohrwarmeubertrager, Heizregister oder Gegenstrom-Schichtwärmeubertrager. Bevorzugt sind Rohr(bündel)wärmeüberträger. Das Medium der Warmetauscher sind vorzugsweise Ol- oder Salzlösungen oder Wasser.

Das aus dem Reaktor austretende Gasgemisch hat eine Temperatur von vorzugsweise ≤ 350°C, besonders bevorzugt ≤ 300°C, noch mehr bevorzugt ≤ 250°C und am meisten bevorzugt ≤ 230. Die Temperatur ist hierbei vorzugsweise ≥ 100°C, besonders bevorzugt ≥ 150°C und am meisten bevorzugt ≥ 180°C. Das austretende Gasgemisch ist im Allgemeinen wärmer als das Gasgemisch, das dem Reaktor zugeführt wird.

Das aus dem Reaktor austretende Gasgemisch enthält Sauerstoff zu Anteilen von vorzugsweise ≤ 1,5 Vol.-%, mehr bevorzugt ≤ 1 Vol.-%, besonders bevorzugt ≤ 0,5 Vol.-%, noch mehr bevorzugt ≤ 0,1 Vol.-% und am meisten bevorzugt ≤ 0,00001 Vol.-%. Der Anteil an Kohlenwasserstoffen beträgt vorzugsweise 55 bis 95 Vol.-%, besonders bevorzugt 60 bis 90 Vol.-% und am meisten bevorzugt 65 bis 80 Vol.-%. In einer alternativen Ausführungsform beträgt der Anteil an Kohlenwasserstoffen vorzugsweise 60 bis 99,99999 Vol.-%, besonders bevorzugt 80 bis 99,99 Vol.-%, noch mehr bevorzugt 90 bis 99,99 Vol.-% und am meisten bevorzugt 95 bis 99,9 Vol.-%. Bei den verbleibenden Gasanteilen handelt es sich um die weiteren Gase ausgewählt aus der Gruppe umfassend Stickstoff, Edelgase, Kohlendioxid, Kohlenmonoxid und Wasser. Die Angaben in Vol.-% beziehen sich auf das Gesamtvolumen des jeweiligen Gasgemisches und addieren sich insgesamt auf 100 Vol.-% auf.

Der Umsatzgrad von Sauerstoff betragt vorzugsweise 50 bis 100 Mol.-%, besonders bevorzugt 80 bis 100 Mol.-%, noch mehr bevorzugt 90 bis 100 Mol.-% und am meisten bevorzugt 95 bis 100 Mol.-%. Der Umsatzgrad bezeichnet hierbei das Molverhältnis aus der Molzahl des Sauerstoffs, die im Reaktor umgesetzt wurde, und der Molzahl des Sauerstoffs, die sich vor bzw. bei Reaktoreintritt im Gasgemisch befunden hat. Der Umsatzgrad bezieht sich hierbei vorzugsweise auf die Umsetzung von Sauerstoff mit Kohlenwasserstoffen zu Kohlendioxid und Wasser.

Das Gasgemisch enthält nach Verlassen des Reaktors vorzugsweise 85 bis 99 Gew.-%, besonders bevorzugt 92 bis 98 Gew.-% und am meisten bevorzugt 95 bis 97 Gew.-% der Kohlenwasserstoffe, die das zu reinigende Gasgemisch vor Eintritt in den Reaktor enthalten hat.

Das Gasgemisch, das den Reaktor verlassen hat, kann direkt seiner Verwertung zugeführt werden. Ethylen enthaltende Gasgemische können beispielsweise für die Herstellung von Vinylchlorid, Acetaldehyd, Ethylenoxid oder insbesondere Vinylacetat eingesetzt werden.

Alternativ kann das Gasgemisch, das den Reaktor verlassen hat, weiter aufgereinigt werden, beispielsweise mittels Destillation, wie Kryodestillation, Membranreingungsverfahren oder Adsorptionsverfahren, wie Waschverfahren. Bevorzugt wird Kohlendioxid mittels alkalischen Wasserwäschern abgetrennt.

Die Durchführung solcher Verfahren zur Verwertung oder Reinigung von Kohlenwasserstoffgasen sind dem Fachmann bekannt.

In einer bevorzugten Ausführungsform wird das Gasgemisch, das den Reaktor verlassen hat, durch einen Gegenstrom-Wärmetauscher geführt. In dem Gegenstrom-Wärmetauscher wird vorzugsweise ein Gasgemisch erwärmt, das dem Reaktor zugeführt wird. Auf diese Weise kann die Energieeffizienz des Verfahrens gesteigert werden.

Das Gasgemisch, das den Gegenstrom-Wärmetauscher verlässt und dem Reaktor zugeführt wird, wird im Gegenstrom-Wärmetauscher um vorzugsweise 20 bis 200°C, besonders bevorzugt 70 bis 180°C und am meisten bevorzugt 100 bis 170°C erwärmt.

In einer weiteren bevorzugten Ausführungsform wird ein Teil des Gasgemisches, das den Reaktor verlassen hat, zu einem Ausgangs-Gasgemisch gegeben und der verbleibende Teil der weiteren Aufreinigung oder Verwertung zugeführt. Der vorgenannte Teil, der zu einem Ausgangs-Gasgemisch gegeben wird, wird im Folgenden auch als Kreisgas bezeichnet. Das Kreisgas wird hierbei vorzugsweise einem Gasgemisch entnommen, das einen oder mehrere Wärmetauscher, insbesondere einen oder mehrere Gegenstrom-Wärmetauscher durchströmt hat. Besonders bevorzugt hat das Gasgemisch einen oder mehrere Gegenstrom-Wärmetauscher und zu einem späteren Zeitpunkt einen oder mehrere weitere Wärmetauscher durchströmt.

Das so erhaltene Gasgemisch aus dem Ausgangs-Gasgemisch und dem Kreisgas kann zur Entfernung von Sauerstoff in einen isotherm betriebenen Reaktor eingeführt werden, in dem der im Gasgemisch enthaltene Sauerstoff in Gegenwart eines oder mehrerer Katalysatoren zumindest teilweise zu Kohlendioxid und Wasser umgesetzt wird, wie oben beschrieben.

Der Anteil des Kreisgases beträgt vorzugsweise 0 Vol.-% bis 95 Vol.-%, besonders bevorzugt 40 Vol.-% bis 85 Vol.-% und am meisten bevorzugt 60 Vol.-% bis 80 Vol.-%, bezogen auf das Gesamtvolumen des den Reaktor verlassenden Gasgemisches.

Die Ausführungsform mit dem Kreisgas hat den Vorteil, dass Ausgangs-Gasgemische mit so hohen Sauerstoff-Gehalten aufgereinigt werden können, die beispielsweise unter den Bedingungen im Reaktor explosiv wären, ohne dass bei der erfindungsgemäßen Vorgehensweise weitere Fremdgasströme oder Sicherheitsmaßnahmen erforderlich werden oder unerwünschte Nebenprodukte gebildet werden. Dadurch wird die Flexibilität des Verfahrens zur Anwendung für unterschiedlichste Gasgemische oder für Gasgemische mit zeitlich variabler Zusammensetzung erheblich gesteigert. Auch beim Anfahren oder Herunterfahren von Anlagen kann auf diese Weise sichergestellt werden, dass die oxidative Reinigung auf sichere Weise erfolgt.

Die Ausgangs-Gasgemische enthalten vorzugsweise ≤ 15 Vol.-%, besonders bevorzugt ≤ 12 Vol.-% und am meisten bevorzugt ≤ 11 Vol.-% Sauerstoff. Bevorzugt sind > 4 Vol.-%, mehr bevorzugt ≥ 5 Vol.-%, besonders bevorzugt > 6,5 Vol.-% und am meisten bevorzugt ≥ 7 Vol.-% Sauerstoff. Der Sauerstoff-Gehalt des Ausgangs-Gasgemisches ist im jeweiligen Verfahren im Allgemeinen größer als der Sauerstoff-Gehalt des Gasgemisches, das in den Reaktor eingeführt wird. Das Ausgangs-Gasgemisch enthalt vorzugsweise 40 bis 95 Vol.-%, besonders bevorzugt 50 bis 90 Vol.-%, noch mehr bevorzugt 60 bis 85 Vol.-% und am meisten bevorzugt 65 bis 80 Vol.-% an einem oder mehreren Kohlenwasserstoffen. Gesattigte Kohlenwasserstoffe, insbesondere Methan oder Ethan, sind vorzugsweise zu 0,1 bis 10 Vol.-% und besonders bevorzugt 3 bis 6 Vol.-% enthalten. Bei den verbleibenden Gasanteilen handelt es sich um die weiteren Gase ausgewählt aus der Gruppe umfassend Stickstoff, Edelgase, Kohlendioxid, Kohlenmonoxid und Wasser. Die Angaben in Vol.-% beziehen sich auf das Gesamtvolumen des jeweiligen Gasgemisches und addieren sich insgesamt auf 100 Vol.-% auf.

Solche Ausgangs-Gasgemische, insbesondere Ethylen-haltige Gasgemische, können beispielsweise bei der Herstellung von Vinylchlorid, Ethylenoxid oder insbesondere bei der katalytischen Herstellung von Vinylacetat aus Ethylen, Essigsäure und Sauerstoff anfallen.

Die Ausgangs-Gasgemische haben Temperaturen von vorzugsweise 0 bis 100°C, besonders bevorzugt 10 bis 80°C und am meisten bevorzugt 20 bis 60°C.

Das Gasgemisch, das nach Mischen des Kreisgases und des Ausgangs-Gasgemisches erhalten wird, enthält vorzugsweise ≥ 50 Vol.-%, mehr bevorzugt 60 bis 93,5 Vol.-%, besonders bevorzugt 65 bis 90 Vol.-%, noch mehr bevorzugt 65 bis 85 Vol.-% und am meisten bevorzugt 70 bis 80 Vol.-% an einem oder mehreren Kohlenwasserstoffen.

Gesättigte Kohlenwasserstoffe, insbesondere Methan oder Ethan, sind hierbei vorzugsweise zu 0,1 bis 10 Vol.-% und besonders bevorzugt 3 bis 6 Vol.-% enthalten. Das Gasgemisch enthält Sauerstoff zu Anteilen von vorzugsweise ≤ 10 Vol.-%, mehr bevorzugt ≤ 6,5 Vol.-%, besonders bevorzugt ≤ 5 Vol.-% und am meisten bevorzugt ≤ 4 Vol.-%. Sauerstoff ist zu Anteilen von vorzugsweise ≥ 2 Vol.-%, besonders bevorzugt ≥ 3 Vol.-% und am meisten bevorzugt ≥ 4 Vol.-% im Gasgemisch enthalten. Bei den verbleibenden Gasanteilen handelt es sich um die weiteren Gase ausgewählt aus der Gruppe umfassend Stickstoff, Edelgase, Kohlendioxid, Kohlenmonoxid und Wasser. Die Angaben in Vol.-% beziehen sich auf das Gesamtvolumen des jeweiligen Gasgemisches und addieren sich insgesamt auf 100 Vol.-% auf.

Gegebenenfalls können an verschiedenen Stellen des erfindungsgemaßen Verfahrens ein oder mehrere Wärmetauscher eingebaut sein, vorzugsweise vor dem Reaktor und/oder nach dem Reaktor. Besonders bevorzugt befindet sich ein Wärmetauscher räumlich zwischen dem Gegenstrom-Wärmetauscher und dem Reaktor. Besonders bevorzugt befindet sich auch ein Wärmetauscher räumlich nach dem Gegenstrom-Wärmetauscher und vor Abtrennung des Kreisgases.

Sämtliche Gasgemische haben im erfindungsgemäßen Verfahren einen Druck von vorzugsweise 3 bis 20 bar_{abs.}, besonders bevorzugt 5 bis 15 bar_{abs}, und am meisten bevorzugt 8 bis 12 bar_{abs.}. Die vorgenannten Drücke herrschen insbesondere im Reaktor und/oder in etwaigen Wärmetauschern und/oder in den Leitungen, durch die die Gasgemische geführt werden. Das Verfahren wird vorzugsweise bei im Wesentlichen konstanten Drücken durchgeführt. Die Druckschwankungen der Gasgemische sind vorzugsweise ≤ 5 bar_{abs.}, besonders bevorzugt ≤ 2 bar_{abs.}, und am meisten bevorzugt ≤ 1 bar_{abs.}. Druckschwankungen bezeichnen hierbei den Druckunterschied zwischen Druckmaxima und Druckminima. Gegebenenfalls können an einer oder mehreren Stollen des erfindungsgemaßen Verfahrens Verdichter eingebaut sein, insbesondere im Falle der Kreisgas-Variante.

In der jeweiligen konkreten Ausführungsform des erfindungsgemäßen Verfahrens werden die Gaszusammensetzung, Druck und Temperatur im Allgemeinen so gewählt, dass die Zündgrenze vorzugsweise gemäß DIN EN 1839-13 unterschritten ist. Die Anwendung der Maßgaben der DIN EN 1839-13 ist dem Fachmann vertraut.

Das erfindungsgemäße Verfahren ermöglicht, Kohlenwasserstoff-haltige Gasgemische vollständig oder zumindest teilweise von Sauerstoff zu befreien. Die Kohlenwasserstoffgase bestehen nach Durchführung der erfindungsgemäßen Reinigung im Allgemeinen im Wesentlichen aus Kohlenwasserstoffen und enthalten im Allgemeinen höchstens noch Spuren an Sauerstoff.

Vorteilhafterweise können mit dem erfindungsgemäßen Verfahren sicherheitstechnisch hochproblematische, hoch Sauerstoffhaltige Kohlenwasserstoff-Gasgemische in leicht handhabbare und ökonomisch interessante, verwertbare Gasgemische überführt werden. Sicherheitstechnisch bedenkliche Bedingungen können hierbei vermieden, und dennoch kann die gewünschte oxidative Reinigung von Kohlenwasserstoff-haltigen Gasgemischen ausgeführt werden. Die Gasgemische können an jeder Stelle des Verfahrens unter Bedingungen, wie Gaszusammensetzung, Druck und Temperatur, gehandhabt werden, die unterhalb der Zündgrenze gemäß DIN EN 1839-13 und damit im sicheren Bereich liegen. Besonders überraschend war auch, dass die oxidative Reinigung im Reaktor bei relativ niedrigen Temperaturen erfolgen konnte. Zudem kann die Bildung von unerwünschten Nebenprodukten zurückgedrängt oder sogar ganz ausgeschlossen werden. Und der im Kohlenwasserstoffgas enthaltene Sauerstoff kann vollständig oder annähernd vollständig zu Kohlendioxid und Wasser umgesetzt werden. Für all diese Vorteile wirkten die Betriebsweise des Reaktors und auch die Führung und Behandlung der Gasströme in synergistischer Weise zusammen.

Die nachfolgenden Beispiele dienen zur weiteren Erläuterung der Erfindung:

### Beispiel 1 (Bsp.1):

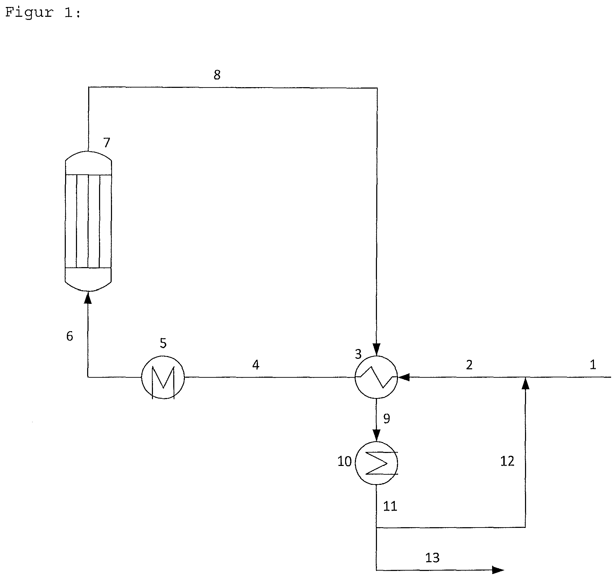

Die oxidative Reinigung des Gasgemisches wurde in einer Anlage gemäß Figur 1 durchgeführt. Angaben zu den Zusammensetzungen, Temperaturen und Drücken des Gasgemisches in den unterschiedlichen Anlagenteilen finden sich in den Tabellen 1 und 2.

Das Gasgemisch 1, d.h. ein Ausgangs-Gasgemisch, wurde mit dem Gasgemisch 12, d.h. einem Kreisgas, gemischt. Das so erhaltene Gasgemisch 2 wurde durch den Gegenstromwärmetauscher 3 durchgeleitet. Hierbei wurde Wärme vom Gasgemisch 8 auf das Gasgemisch 2 übertragen. Das so erwärmte Gasgemisch 4 wurde zur weiteren Temperierung durch den mit überhitztem Wasserdampf betriebenen Wärmetauscher 5 geführt und als weiter erwärmtes Gasgemisch 6 in den Rohrbündelreaktor 7 eingebracht. Der Rohrbündelreaktor 7 war mit einem geträgerten Palladium-Gold-Festbettkatalysator beschickt und wurde mittels Siedewasserkühlung isotherm betrieben.

Der Sauerstoffumsatz im Rohrbündelreaktor 7 betrug 97 mol.-%, bezogen auf den in den Reaktor eingebrachten Sauerstoff.

Das aus dem Rohrbündelreaktor 7 austretende Gasgemisch 8 kühlte sich beim Durchströmen des Gegenstromwärmetauschers 3 ab und wurde als Gasgemisch 9 zur weiteren Abkühlung durch den mit Kühlwasser betriebenen Wärmetauscher 10 geleitet. Hierbei kondensierte Wasser aus.

77 Mol.-% des Gasgemisches 11, bezogen auf den Molenstrom von Gasgemisch 11, wurden als Kreisgas 12 mit dem Gasgemisch 1 zum Gasgemisch 2 gemischt und damit in das Verfahren zurückgeführt. Die verbleibenden 23 Mol.-% des Gasgemisches 11 wurden der Verwertung zugeführt.

Das aufgereinigte Gasgemisch 13 hatte die identische Zusammensetzung wie das Gasgemisch 11 und enthielt somit nur noch Spuren an Sauerstoff. Die Bildung etwaiger Nebenprodukte, wie Kohlenmonoxid oder Ethylenoxid, wurde nicht beobachtet. Auch eine Hydrierung von Ethylen zu Ethan trat nicht auf.

**Tabelle 1: Zusammensetzungen der Gasgemische des Beispiels 1:**

| | Ethylen | Sauerstoff | Kohlendioxid | Ethan, Methan | Inerte^{a)} |
|---|---|---|---|---|---|
| | [Vol. %] | [Vol.%] | [Vol.%] | [Vol.%] | [Vol.%] |
| Gasgemisch 1 | 76,8 | 9,1 | 1,7 | 5,4 | 7,2 |
| Gasgemisch 2 | 78,2 | 2,2 | 6,6 | 5,5 | 7,5 |
| Gasgemisch 8 | 77,43 | 0,07 | 8,1 | 5,5 | 8,9 |
| Gasgemisch 11 | 78,53 | 0,07 | 8,2 | 5,6 | 7,6 |

| | | | | | |
|---|---|---|---|---|---|
| a) Edelgase, Stickstoff und Wasser. | | | | | |

**Tabelle 2: Temperatur und Druck der Gasgemische der Beispiele:**

| | | Bsp.1 | Bsp.2 | Bsp.3 | Bsp.4 | Bsp.5 |
|---|---|---|---|---|---|---|
| Gasgemisch 1 | Temperatur [°C] | 25 | 50 | 30 | 25 | 25 |
| | Druck [bar_{abs.}] | 10,5 | 10,5 | 9,5 | 10,5 | 10,5 |
| Gasgemisch 2 | Temperatur [°C] | 28 | 40 | 30 | 29 | 29 |
| | Druck [bar_{abs.}] | 10,5 | 10,5 | 9,5 | 10,5 | 10,5 |
| Gasgemisch 4 | Temperatur [°C] | 175 | 175 | 175 | 190 | 190 |
| | Druck [bar_{abs.}] | 10,45 | 10,45 | 9,45 | 10,45 | 10,45 |
| Gasgemisch 6 | Temperatur [°C] | 195 | 195 | 195 | 220 | 220 |
| | Druck [bar_{abs.}] | 10,42 | 10,42 | 9,42 | 10,42 | 10,42 |
| Gasgemisch 8 | Temperatur [°C] | 215 | 205 | 215 | 230 | 230 |
| | Druck [bar_{abs.}] | 10,32 | 10,32 | 9,32 | 10,32 | 10,32 |
| Gasgemisch 9 | Temperatur [°C] | 78 | 78 | 78 | 80 | 80 |
| | Druck [bar_{abs.}] | 10,29 | 10,29 | 9,29 | 10,29 | 10,29 |
| Gasgemisch 11 | Temperatur [°C] | 25 | 25 | 25 | 25 | 25 |
| | Druck [bar_{abs.}] | 10,29 | 10,29 | 9,29 | 10,29 | 10,29 |
| Gasgemisch 12 | Temperatur [°C] | 25 | 25 | 25 | 25 | 25 |
| | Druck [bar_{abs.}] | 10,83 | 10,83 | 9,83 | 10,83 | 10,83 |
| Gasgemisch 13 | Temperatur [°C] | 25 | 25 | 25 | 25 | 25 |
| | Druck [bar_{abs.}] | 10,83 | 10,83 | 9,83 | 10,83 | 10,83 |

### Beispiel 2 (Bsp.2):

Beispiel 2 wurde identisch ausgeführt wie Beispiel 1, mit folgenden Unterschieden:
Das Gasgemisch 1, d.h. das Ausgangs-Gasgemisch, hatte eine andere Zusammensetzung, wie in Tabelle 3 angegeben.

Der Sauerstoffumsatz im Rohrbündelreaktor 7 betrug 84 mol.-%, bezogen auf den in den Reaktor eingebrachten Sauerstoff.

47 Mol.-% des Gasgemisches 11, bezogen auf den Molenstrom von Gasgemisch 11, wurden als Kreisgas 12 in das Verfahren zurückgeführt. Die verbleibenden 53 Mol.-% des Gasgemisches 11 wurden der Verwertung zugeführt.

Weitere Angaben zu den Zusammensetzungen, Temperaturen und Drücken des Gasgemisches an den unterschiedlichen Stellen des Verfahrens finden sich in den Tabellen 2 und 3.

**Tabelle 3: Zusammensetzungen der Gasgemische des Beispiels 2:**

| | Ethylen | Sauerstoff | Kohlendioxid | Ethan, Methan | Inerte^{a)} |
|---|---|---|---|---|---|
| | [Vol.%] | [Vol.%] | [Vol.%] | [Vol.%] | [Vol.%] |
| Gasgemisch 1 | 75,0 | 11,1 | 2,0 | 5,2 | 6,7 |
| Gasgemisch 2 | 75,8 | 6,5 | 5,0 | 5,4 | 7,3 |
| Gasgemisch 8 | 74,0 | 1,0 | 8,7 | 5,4 | 10,9 |
| Gasgemisch 11 | 76,8 | 1,0 | 9,0 | 5,6 | 7,6 |

| | | | | | |
|---|---|---|---|---|---|
| a) Edelgase, Stickstoff und Wasser. | | | | | |

### Beispiel 3 (Bsp.3):

Beispiel 3 wurde identisch ausgeführt wie Beispiel 1, mit folgenden Unterschieden:
Das Gasgemisch 1, d.h. das Ausgangs-Gasgemisch, hatte eine andere Zusammensetzung, wie in Tabelle 4 angegeben.

Der Sauerstoffumsatz im Rohrbündelreaktor 7 betrug 95 mol.-%, bezogen auf den in den Reaktor eingebrachten Sauerstoff.

Dem Gasgemisch 11 wurde kein Kreisgas 12 entnommen; d.h. das Gasgemisch 11 wurde vollständig der Verwertung zugeführt. Weitere Angaben zu den Zusammensetzungen, Temperaturen und Drücken des Gasgemisches an den unterschiedlichen Stellen des Verfahrens finden sich in den Tabellen 2 und 4.

**Tabelle 4: Zusammensetzungen der Gasgemische des Beispiels 3:**

| | Ethylen | Sauerstoff | Kohlendioxid | Ethan, Methan | Inerte^{a)} |
|---|---|---|---|---|---|
| | [Vol.%] | [Vol.%] | [Vol.%] | [Vol.%] | [Vol.%] |
| Gasgemisch 1 | 81,2 | 5,0 | 1,6 | 5,2 | 7,0 |
| Gasgemisch 2 | 81,2 | 5,0 | 1,6 | 5,2 | 7,0 |
| Gasgemisch 8 | 79,6 | 0,3 | 4,8 | 5,2 | 10,1 |
| Gasgemisch 11 | 82,2 | 0,3 | 4,9 | 5,4 | 7,3 |

| | | | | | |
|---|---|---|---|---|---|
| a) Edelgase, Stickstoff und Wasser. | | | | | |

### Beispiel 4 (Bsp. 4): (nicht erfindungsgemäß)

Beispiel 4 wurde identisch ausgeführt wie Beispiel 1, mit folgenden Unterschieden:
Das Gasgemisch 1, d.h. das Ausgangs-Gasgemisch, hatte eine andere Zusammensetzung, wie in Tabelle 5 angegeben.

Der Wärmetauscher 5 wurde mit Wärmeträgeröl betrieben. Der Rohrbündelreaktor 7 war an Stelle des Palladium-Gold-Katalysators mit einem geträgerten Palladium-Festbettkatalysator beschickt.

Der Sauerstoffumsatz im Rohrbündelreaktor 7 betrug 40 mol.-%, bezogen auf den in den Reaktor eingebrachten Sauerstoff. 92 Mol.-% des Gasgemisches 11, bezogen auf den Molenstrom von Gasgemisch 11, wurden als Kreisgas 12 in das Verfahren zurückgeführt. Die verbleibenden 8 Mol.-% des Gasgemisches 11 wurden der Verwertung zugeführt.

Weitere Angaben zu den Zusammensetzungen, Temperaturen und Drücken des Gasgemisches an den unterschiedlichen Stellen des Verfahrens finden sich in den Tabellen 2 und 4.

**Tabelle 5: Zusammensetzungen der Gasgemische des Beispiels 4:**

| | Ethylen | Sauerstoff | Kohlendioxid, Kohlenmonoxid | Ethan, Methan | Inerte^{a)} |
|---|---|---|---|---|---|
| | [Vol.%] | [Vol.%] | [Vol.%] | [Vol.%] | [Vol.%] |
| Gasgemisch 1 | 77,7 | 8,2 | 1,7^{b)} | 5,4 | 7,0 |
| Gasgemisch 2 | 79,1 | 1,7 | 6,3 | 5,5 | 7,4 |
| Gasgemisch 8 | 78,9 | 1,0 | 6,8 | 5,5 | 7,8 |
| Gasgemisch 11 | 79,3 | 1,0 | 6,8 | 5,5 | 7,4 |

| | | | | | |
|---|---|---|---|---|---|
| a) Edelgase, Stickstoff und Wasser; b) kein Kohlenmonoxid im Ausgangs-Gas 1 enthalten. | | | | | |

### Beispiel 5 (Bsp. 5): (nicht erfindungsgemäß)

Beispiel 5 wurde identisch ausgeführt wie Beispiel 4, mit folgenden Unterschieden:
Das Gasgemisch 1, d.h. das Ausgangs-Gasgemisch, hatte eine andere Zusammensetzung, wie in Tabelle 5 angegeben.

Der Sauerstoffumsatz im Rohrbündelreaktor 7 betrug 40 mol.-%, bezogen auf den in den Reaktor eingebrachten Sauerstoff.

91 Mol.-% des Gasgemisches 11, bezogen auf den Molenstrom von Gasgemisch 11, wurden als Kreisgas 12 in das Verfahren zurückgeführt. Die verbleibenden 9 Mol.-% des Gasgemisches 11 wurden der Verwertung zugeführt.

Weitere Angaben zu den Zusammensetzungen, Temperaturen und Drücken des Gasgemisches an den unterschiedlichen Stellen des Verfahrens finden sich in den Tabellen 2 und 4.

Bei Einsatz eines geträgerten Platin-Festbettkatalysators an Stelle des Palladium-Katalysators in den Verfahren der Beispiele 4 und 5 wurden die Ergebnisse der Beispiele 4 und 5 im Wesentlichen reproduziert.

**Tabelle 6: Zusammensetzungen der Gasgemische des Beispiels 5:**

| | Ethylen | Sauerstoff | Kohlendioxid, Kohlenmonoxid | Ethan, Methan | Inerte^{a)} |
|---|---|---|---|---|---|
| | [Vol.%] | [Vol.%] | [Vol.%] | [Vol.%] | [Vol.%] |
| Gasgemisch 1 | 80,9 | 5,0 | 1,7^{b)} | 5,2 | 7,2 |
| Gasgemisch 2 | 81,9 | 1,0 | 4,5 | 5,4 | 7,2 |
| Gasgemisch 8 | 81,7 | 0,6 | 4,7 | 5,4 | 7,5 |
| Gasgemisch 11 | 82,0 | 0,6 | 4,7 | 5,4 | 7,3 |

| | | | | | |
|---|---|---|---|---|---|
| a) Edelgase, Stickstoff und Wasser; b) kein Kohlenmonoxid im Ausgangs-Gas 1 enthalten. | | | | | |

## Patentansprüche

1. Verfahren zur Entfernung von Sauerstoff aus Kohlenwasserstoff-haltigen Gasgemischen, **dadurch gekennzeichnet, dass** ein Kohlenwasserstoff-haltiges Gasgemisch enthaltend ≥ 50 Vol.-% an einem oder mehreren Kohlenwasserstoffen, 2 bis 10 Vol.-% Sauerstoff und gegebenenfalls ein oder mehrere Gase aus der Gruppe umfassend Stickstoff, Edelgase, Kohlendioxid, Kohlenmonoxid und Wasser,
in einen isotherm betriebenen Reaktor eingeführt wird,
in dem der im Kohlenwasserstoff-haltigen Gasgemisch enthaltene Sauerstoff in Gegenwart eines oder mehrerer Katalysatoren zumindest teilweise zu Kohlendioxid und Wasser umgesetzt wird,
wobei das Kohlenwasserstoff-haltige Gasgemisch keinen Wasserstoff enthält und dem Kohlenwasserstoff-haltigen Gasgemisch kein Wasserstoff zugesetzt wird, und
wobei ein oder mehrere Katalysatoren ausgewählt werden aus der Gruppe umfassend Palladium/Gold, Platin/Gold und deren Salze,
wobei sich die Angaben in Vol.-% auf das Gesamtvolumen des in den Reaktor eingeführten Kohlenwasserstoff-haltigen Gasgemisches beziehen und sich insgesamt auf 100 Vol.-% aufaddieren.

2. Verfahren zur Entfernung von Sauerstoff aus Kohlenwasserstoff-haltigen Gasgemischen nach Anspruch 1, **dadurch gekennzeichnet, dass** kein Sauerstoff als weiteres Gas in den Reaktor eingeführt wird.

3. Verfahren zur Entfernung von Sauerstoff aus Kohlenwasserstoff-haltigen Gasgemischen nach Anspruch 1 oder 2, **dadurch gekennzeichnet, dass** die Temperatur des aus dem Reaktor austretenden Gasgemisches sich von der Temperatur des in den Reaktor eintretenden Kohlenwasserstoff-haltigen Gasgemisches um ≤ 50°C unterscheidet.

4. Verfahren zur Entfernung von Sauerstoff aus Kohlenwasserstoff-haltigen Gasgemischen nach Anspruch 1 bis 3, **dadurch gekennzeichnet, dass** das Kohlenwasserstoff-haltige Gasgemisch bei Eintritt in den Reaktor eine Temperatur von ≤ 300°C und ≥ 100°C hat.

5. Verfahren zur Entfernung von Sauerstoff aus Kohlenwasserstoff-haltigen Gasgemischen nach Anspruch 1 bis 4, **dadurch gekennzeichnet, dass** das aus dem Reaktor austretende Gasgemisch eine Temperatur von ≤ 350°C und ≥ 100°C hat.

6. Verfahren zur Entfernung von Sauerstoff aus Kohlenwasserstoff-haltigen Gasgemischen nach Anspruch 1 bis 5, **dadurch gekennzeichnet, dass** das aus dem Reaktor austretende Gasgemisch ≤ 1,5 Vol.-% Sauerstoff, 55 bis 99,99999 Vol.-% an einem oder mehreren Kohlenwasserstoffen und gegebenenfalls ein oder mehrere weitere Gase ausgewählt aus der Gruppe umfassend Stickstoff, Edelgase, Kohlendioxid, Kohlenmonoxid und Wasser enthält, wobei sich die Angaben in Vol.-% auf das Gesamtvolumen des jeweiligen Gasgemisches beziehen und sich insgesamt auf 100 Vol.-% aufaddieren.

7. Verfahren zur Entfernung von Sauerstoff aus Kohlenwasserstoff-haltigen Gasgemischen nach Anspruch 1 bis 6, **dadurch gekennzeichnet, dass** der Umsatzgrad von Sauerstoff im Reaktor 50 bis 100 Mol.-% beträgt.

8. Verfahren zur Entfernung von Sauerstoff aus Kohlenwasserstoff-haltigen Gasgemischen nach Anspruch 1 bis 7, **dadurch gekennzeichnet, dass** das den Reaktor verlassende Gasgemisch 85 bis 99 Gew.-% der Kohlenwasserstoffe enthält, die das zu reinigende, in den Reaktor eingeführte Kohlenwasserstoff-haltige Gasgemisch enthalten hat.

9. Verfahren zur Entfernung von Sauerstoff aus Kohlenwasserstoff-haltigen Gasgemischen nach Anspruch 1 bis 8, **dadurch gekennzeichnet, dass** das Gasgemisch, das den Reaktor verlassen hat, vollständig oder teilweise durch einen Gegenstrom-Wärmetauscher geführt wird, in dem ein Kohlenwasserstoff-haltiges Gasgemisch erwärmt wird, das nach Verlassen des Gegenstrom-Wärmetauschers dem Reaktor zugeführt wird.

10. Verfahren zur Entfernung von Sauerstoff aus Kohlenwasserstoff-haltigen Gasgemischen nach Anspruch 1 bis 9, **dadurch gekennzeichnet, dass** das Gasgemisch, das den Reaktor verlassen hat, teilweise der weiteren Aufreinigung oder Verwertung zugeführt wird und zum verbleibenden Teil zu einem oder mehreren Ausgangs-Gasgemischen gegeben wird,
wobei Ausgangs-Gasgemische ≤ 15 Vol.-% Sauerstoff, 40 bis 95 Vol.-% an einem oder mehreren Kohlenwasserstoffen und gegebenenfalls ein oder mehrere weitere Gase ausgewählt aus der Gruppe umfassend Stickstoff, Edelgase, Kohlendioxid, Kohlenmonoxid und Wasser enthalten, wobei sich die Angaben in Vol.-% auf das Gesamtvolumen des jeweiligen Gasgemisches beziehen und sich insgesamt auf 100 Vol.-% aufaddieren.

## Claims

1. Process for removing oxygen from hydrocarbon-containing gas mixtures, **characterized in that**
a hydrocarbon-containing gas mixture containing ≥ 50 % by volume of one or more hydrocarbons, from 2 to 10 % by volume of oxygen and optionally one or more gases from the group consisting of nitrogen, noble gases, carbon dioxide, carbon monoxide and water
is introduced into an isothermally operated reactor in which the oxygen present in the hydrocarbon-containing gas mixture is at least partly converted into carbon dioxide and water in the presence of one of more catalysts,
wherein the hydrocarbon-containing gas mixture does not contain any hydrogen and no hydrogen is added to the hydrocarbon-containing gas mixture and
one or more catalysts are selected from the group consisting of palladium/gold, platinum/gold and salts thereof,
where the figures in % by volume are based on the total volume of the hydrocarbon-containing gas mixture introduced into the reactor and add up to a total of 100% by volume.

2. Process for removing oxygen from hydrocarbon-containing gas mixtures according to Claim 1, **characterized in that** no oxygen is introduced as further gas into the reactor.

3. Process for removing oxygen from hydrocarbon-containing gas mixtures according to Claim 1 or 2, **characterized in that** the temperature of the gas mixture exiting from the reactor differs from the temperature of the hydrocarbon-containing gas mixture entering the reactor by ≤ 50°C.

4. Process for removing oxygen from hydrocarbon-containing gas mixtures according to any of Claims 1 to 3, **characterized in that** the hydrocarbon-containing gas mixture has a temperature of from ≤ 300°C to ≥ 100°C on entering the reactor.

5. Process for removing oxygen from hydrocarbon-containing gas mixtures according to any of Claims 1 to 4, **characterized in that** the gas mixture leaving the reactor has a temperature of from ≤ 350°C to ≥ 100°C.

6. Process for removing oxygen from hydrocarbon-containing gas mixtures according to any of Claims 1 to 5, **characterized in that** the gas mixture leaving the reactor contains ≤ 1.5% by volume of oxygen, from 55 to 99.99999% by volume of one or more hydrocarbons and optionally one or more further gases selected from the group consisting of nitrogen, noble gases, hydrogen, carbon dioxide, carbon monoxide and water, where the figures in % by volume are based on the total volume of the respective gas mixture and add up to a total of 100% by volume.

7. Process for removing oxygen from hydrocarbon-containing gas mixtures according to any of Claims 1 to 6, **characterized in that** the degree of conversion of oxygen in the reactor is from 50 to 100 mol%.

8. Process for removing oxygen from hydrocarbon-containing gas mixtures according to any of Claims 1 to 7, **characterized in that** the gas mixture leaving the reactor contains from 85 to 99% by weight of the hydrocarbons which were present in the hydrocarbon-containing gas mixture to be purified which was introduced into the reactor.

9. Process for removing oxygen from hydrocarbon-containing gas mixtures according to any of Claims 1 to 8, **characterized in that** the gas mixture which has left the reactor is conveyed in its entirety or in part through a countercurrent heat exchanger in which a hydrocarbon-containing gas mixture which, after leaving the countercurrent heat exchanger, is fed to the reactor is heated.

10. Process for removing oxygen from hydrocarbon-containing gas mixtures according to any of Claims 1 to 9, **characterized in that** the gas mixture which has left the reactor is partly fed to further purification or utilization and the remaining part is added to one or more starting gas mixtures,
wherein starting gas mixtures contain ≤ 15% by volume of oxygen, from 40 to 95% by volume of one or more hydrocarbons and optionally one or more further gases selected from the group consisting of nitrogen, noble gases, carbon dioxide, carbon monoxide and water, where the figures in % by volume are based on the total volume of the respective gas mixture and add up to a total of 100% by volume.

## Revendications

1. Procédé d'élimination d'oxygène de mélanges gazeux contenant des hydrocarbures, **caractérisé en ce qu'**un mélange gazeux contenant des hydrocarbures contenant ≥ 50 % en volume d'un ou de plusieurs hydrocarbures, 2 à 10 % en volume d'oxygène et éventuellement un ou plusieurs gaz du groupe comprenant l'azote, les gaz nobles, le dioxyde de carbone, le monoxyde de carbone et l'eau,
est introduit dans un réacteur exploité de manière isotherme,
dans lequel l'oxygène contenu dans le mélange gazeux contenant des hydrocarbures est au moins partiellement transformé en présence d'un ou de plusieurs catalyseurs en dioxyde de carbone et eau, et
le mélange gazeux contenant des hydrocarbures ne contenant pas d'hydrogène et de l'hydrogène n'étant pas ajouté au mélange gazeux contenant des hydrocarbures, et un ou plusieurs catalyseurs étant choisis dans le groupe comprenant le palladium/or, le platine/or et leurs sels, les indications en % en volume se rapportant au volume total du mélange gazeux contenant des hydrocarbures introduit dans le réacteur et leur somme totale étant de 100 % en volume.

2. Procédé d'élimination d'oxygène de mélanges gazeux contenant des hydrocarbures selon la revendication 1, **caractérisé en ce que** de l'oxygène n'est pas introduit dans le réacteur en tant que gaz supplémentaire.

3. Procédé d'élimination d'oxygène de mélanges gazeux contenant des hydrocarbures selon la revendication 1 ou 2, **caractérisé en ce que** la température du mélange gazeux sortant du réacteur diffère de la température du mélange gazeux contenant des hydrocarbures entrant dans le réacteur de ≤ 50 °C.

4. Procédé d'élimination d'oxygène de mélanges gazeux contenant des hydrocarbures selon les revendications 1 à 3, **caractérisé en ce que** le mélange gazeux contenant des hydrocarbures présente lors de l'entrée dans le réacteur une température ≤ 300 °C et ≥ 100 °C.

5. Procédé d'élimination d'oxygène de mélanges gazeux contenant des hydrocarbures selon les revendications 1 à 4, **caractérisé en ce que** le mélange gazeux sortant du réacteur présente une température ≤ 350 °C et ≥ 100 °C.

6. Procédé d'élimination d'oxygène de mélanges gazeux contenant des hydrocarbures selon les revendications 1 à 5, **caractérisé en ce que** le mélange gazeux sortant du réacteur contient ≤ 1,5 % en volume d'oxygène, 55 à 99,99999 % en volume d'un ou de plusieurs hydrocarbures et éventuellement un ou plusieurs gaz supplémentaires choisis dans le groupe comprenant l'azote, les gaz nobles, le dioxyde de carbone, le monoxyde de carbone et l'eau, les indications en % en volume se rapportant au volume total du mélange gazeux en question et leur somme totale étant de 100 % en volume.

7. Procédé d'élimination d'oxygène de mélanges gazeux contenant des hydrocarbures selon les revendications 1 à 6, **caractérisé en ce que** le degré de conversion de l'oxygène dans le réacteur est de 50 à 100 % en moles.

8. Procédé d'élimination d'oxygène de mélanges gazeux contenant des hydrocarbures selon les revendications 1 à 7, **caractérisé en ce que** le mélange gazeux quittant le réacteur contient 85 à 99 % en poids des hydrocarbures que le mélange gazeux contenant des hydrocarbures à purifier introduit dans le réacteur contenait.

9. Procédé d'élimination d'oxygène de mélanges gazeux contenant des hydrocarbures selon les revendications 1 à 8, **caractérisé en ce que** le mélange gazeux qui a quitté le réacteur est acheminé en totalité ou en partie dans un échangeur de chaleur à contre-courant, dans lequel un mélange gazeux contenant des hydrocarbures est chauffé et introduit dans le réacteur à la sortie de l'échangeur de chaleur à contre-courant.

10. Procédé d'élimination d'oxygène de mélanges gazeux contenant des hydrocarbures selon les revendications 1 à 9, **caractérisé en ce que** le mélange gazeux qui a quitté le réacteur est introduit en partie dans une purification supplémentaire ou une valorisation, et la partie restante est ajoutée à un ou plusieurs mélanges gazeux initiaux, les mélanges gazeux initiaux contenant ≤ 15 % en volume d'oxygène, 40 à 95 % en volume d'un ou de plusieurs hydrocarbures et éventuellement un ou plusieurs gaz supplémentaires du groupe comprenant l'azote, les gaz nobles, le dioxyde de carbone, le monoxyde de carbone et l'eau, les indications en % en volume se rapportant au volume total du mélange gazeux en question et leur somme totale étant de 100 % en volume.
